Europäisches Patentamt

European Patent Office

Office européen des brevets

(19)

(11) Numéro de publication: **0 108 669**
**A1**

(12) **DEMANDE DE BREVET EUROPEEN**

(21) Numéro de dépôt: **83401985.3**

(22) Date de dépôt: **12.10.83**

(51) Int. Cl.³: **B 26 B 19/38**
**B 21 D 53/64**

(30) Priorité: **22.10.82 FR 8217681**

(43) Date de publication de la demande:
**16.05.84 Bulletin 84/20**

(84) Etats contractants désignés:
**AT BE DE FR GB IT NL**

(71) Demandeur: **SEB S.A.**

**F-21260 Selongey(FR)**

(72) Inventeur: **Schwob, Pierre**
**55 Avenue des Frères Lumière**
**F-69008 Lyon(FR)**

(74) Mandataire: **Bouju, André**
**38 Avenue de la Grande Armée**
**F-75017 Paris(FR)**

(54) **Procédé pour fabriquer une grille pour rasoir électrique et grille ainsi obtenue.**

(57) Le procédé pour fabriquer une grille pour tête de rasoir électrique comprend le découpage dans une feuille métallique (1) d'un flan (2) sensiblement rectangulaire présentant les ouvertures (4) de la grille, le pliage de ce flan de façon à obtenir une section transversale en U, les ouvertures (4) de la grille s'étendant sur la base de cette section puis la rectification et le polissage de la grille.

Le grille est découpée dans une feuille d'acier inoxydable, puis après pliage de celle-ci, on rectifie seulement la face intérieure (12) de la base comportant les ouvertures et on polit directement la face extérieure de cette base sans la rectifier, ni la chromer.

Utilisation notamment pour têtes de coupe de rasoirs électriques ou appareils à raser analogues.

FIG. 4B

1

"Procédé pour fabriquer une grille pour rasoir électrique et grille ainsi obtenue"

La présente invention concerne un procédé pour fabriquer une grille de coupe de rasoir électrique ou appareil à raser analogue.

L'invention vise également la grille obtenue notamment selon le procédé précité.

L'invention s'applique aux appareils à raser, notamment à actionnement électrique, du genre comportant une grille qui coopère avec des couteaux disposés contre celle-ci et animés d'un mouvement alternatif de translation ou d'un mouvement de rotation.

En pénétrant dans les ouvertures de la grille, les poils sont coupés par effet de ciseau entre les couteaux et les bords intérieurs des ouvertures de la grille.

On connaît des tête de coupe à mouvement alternatif des couteaux dans lesquelles la surface de contact de la grille avec la peau présente une section transversale constituée par deux U jumelés séparés par une zone en creux, le couteau lui-même étant constitué par une grille tubulaire de section carrée dont la zone de coupe présente un profil homologue de celui de la grille. On connaît également des grilles de section transversale en forme de V tronqué dont la base plane est en contact avec la peau, le couteau étant lui-même constitué par une grille de section transversale en U.

Ces grilles sont obtenues par découpe, pliage ou emboutissage de flans en acier au carbone, cette opération étant suivie d'une trempe et d'un revenu.

Différemment les grilles de têtes rotatives sont le plus souvent usinées.

Toutefois, quelles qu'aient été les premières opérations de fabrication, ces grilles subissent une opération de mise à épaisseur de leur zone active de coupe (en général 0,07 mm) réalisée par enlèvement de métal par rectification à la meule sur leurs faces intérieure et extérieure, suivie d'un polissage de la face extérieure.

Ces opérations sont complétées par un chromage brillant.

De tels procédés de fabrication présentent plusieurs inconvénients :

- ils sont onéreux du fait qu'ils exigent une double opération de rectification intérieure et extérieure ; la rectification de la face extérieure crée des formes anguleuses que le polissage doit adoucir, ce qui complique cette dernière opération ;

- il subsiste après polissage des bavures dues à l'usinage qui sont ensuite épaissies et fixées par le chromage ce qui réduit l'efficacité de la coupe;

- la rugosité des flancs de découpe est augmentée par le chromage ce qui rend plus difficile le glissement des poils dans les ouvertures de la grille;

- on constate une usure relativement rapide des couteaux à cause des bavures subsistant sur la surface de contact de la grille.

Le but de la présente invention est de remédier aux inconvénients précités en présentant un procédé de fabrication des grilles pour têtes de rasoir qui soit à la fois plus économique que ceux utilisés précédemment et qui permette d'obtenir une grille plus performante.

Le procédé visé par l'invention pour fabriquer une grille pour tête de rasoir électrique comprend le découpage d'une feuille en acier inoxydable pour constituer un flan sensiblement rectangulaire présentant les ouvertures de la grille, le pliage de ce flan de façon à obtenir une grille de section transversale en U, les ouvertures de la grille s'étendant sur la base de cette section, puis la rectification et le polissage de la surface de la grille.

Suivant l'invention, ce procédé est caractérisé par les étapes suivantes:

- après pliage du flan on rectifie seulement la face intérieure de la base comportant les ouvertures, pour amener la grille à son épaisseur normale d'utilisation,

- on polit directement la face extérieure de cette base sans la rectifier, ni la chromer.

Le procédé selon l'invention diffère de celui connu décrit précédemment notamment par la suppression de deux étapes, à savoir la rectification de la face extérieure de la base de la grille et le chromage de la grille, et par une simplification du polissage qui ne nécessite plus un adoucissage de la surface extérieure rendue anguleuse par la rectification.

La suppression de ces étapes compense largement l'utilisation d'un acier inoxydable plus onéreux qu'un acier au carbone. D'autre part, étant donné que l'acier inoxydable présente une dureté supérieure à l'acier au carbone précédemment utilisé, il est possible de partir d'une feuille plus mince. Il résulte de l'ensemble de ces considérations que le bilan économique du procédé selon l'invention est nettement positif.

L'expérience montre de plus que ce procédé permet également d'obtenir de manière surprenante de meilleures performances de la tête de coupe ne pouvant être prévues du seul fait de l'utilisation d'acier inoxydable. En effet, si de manière analogue aux procédés antérieurs l'opération de polissage fait tomber une partie des bavures et diminue la rugosité des flancs de découpe, la suppression du chromage fait que dès les premières utilisations, les bavures restantes se détachent d'elles-mêmes des arêtes tranchantes de la grille par l'effet des couteaux, en procurant très rapidement l'équivalent d'un rodage de la tête du rasoir.

Selon une version préférée du procédé selon l'invention, on forme sur la face extérieure de la base de la grille pliée en U un méplat embouti vers l'intérieur de la grille, on rectifie la face intérieure de ce méplat embouti et on polit la face extérieure de la grille y compris la face extérieure du méplat embouti.

Cette particularité du procédé permet de rectifier seulement la partie active de la grille c'est-à-dire une partie de la zone des ouvertures en laissant au cadre formé par le reste de la grille sa pleine épaisseur d'origine et sa rigidité.

Selon un autre aspect de l'invention, la grille pour tête de rasoir électrique comprenant une feuille de métal sensiblement rectangulaire pliée de façon à présenter une section transversale sensiblement en U, la base du U de cette grille présentant des ouvertures est caractérisée en ce qu'elle comprend sur cette base une cavité emboutie vers l'intérieur de la grille qui présente un fond plat, la face intérieure de ce fond comportant un évidement rectifié dont la largeur est adaptée à celle des couteaux tandis que la face extérieure de la grille y compris celle de la cavité emboutie est uniquement polie.

D'autres particularités et avantages de l'invention apparaîtront encore dans la description ci-après.

Aux dessins annexés donnés à titre d'exemples non limitatifs:

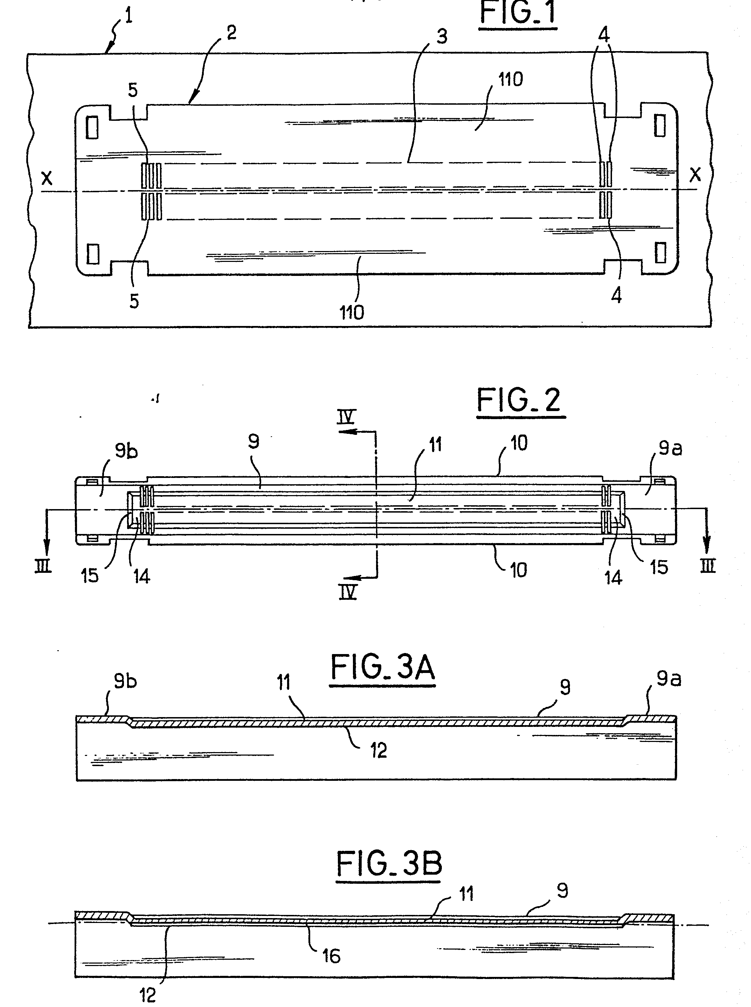

. la figure 1 est une vue agrandie en plan du plan découpé de la grille avec ses ouvertures illustrant la première étape du procédé selon l'invention;

. la figure 2 est une vue de dessus de la grille après pliage en U du flan et emboutissage du méplat;

. la figure 3A est une vue en coupe longitudinale de la grille suivant le plan III-III de la figure 2 avant rectification et la figure 3B représente cette même vue après rectification;

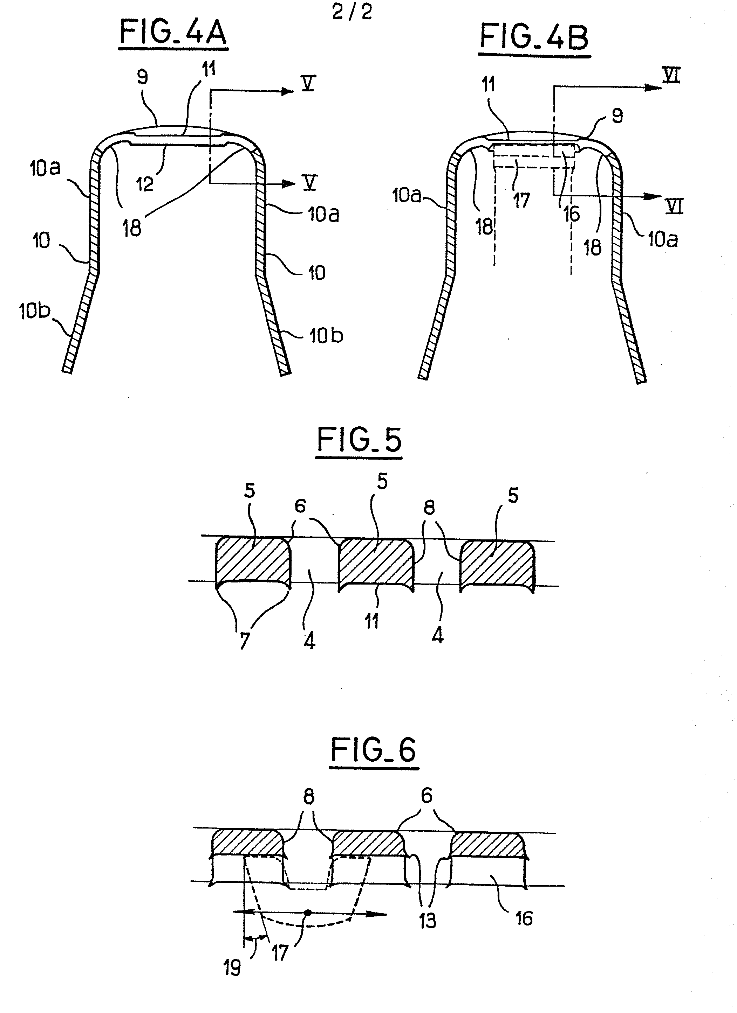

. la figure 4A est une vue en coupe transversale suivant le plan IV-IV de la figure 2 avant rectification et la figure 4B représente cette même vue en coupe après rectification sur laquelle on a représenté en coupe, en pointillé, un couteau de la tête de rasoir;

. la figure 5 est une vue en coupe longitudinale partielle à plus grande échelle suivant le plan V-V de la figure 4A montrant les barreaux de la grille avant rectification;

. la figure 6 est une vue en coupe longitudinale partielle à plus grande échelle suivant le plan VI-VI de la figure 4B montrant les barreaux de la grille après rectification sur laquelle on a également représenté en coupe transversale un couteau coopérant avec la grille.

On décrira tout d'abord le procédé de réalisation de la grille pour tête de coupe de rasoir électrique en référence aux figures 1 à 6.

On découpe tout d'abord, comme indiqué à la figure 1, dans une feuille d'acier inoxydable 1, un flan sensiblement rectangulaire 2 par exemple d'épaisseur égale à 0,2 mm par exemple à l'aide d'une presse à découper.

Ce flan 2 est ensuite repris pour découpe pas à pas à l'aide d'un outil à suivre sur une zone centrale longitudinale d'une double série d'ouvertures rectangulaires étroites 4 situées de part et d'autre de l'axe de symétrie longitudinal X-X de ce flan 2. On forme ainsi une double série de barreaux rectangulaires étroits 5 de largeur voisine de celle des ouvertures 4. Cette opération entraîne à la partie supérieure des barreaux 5 la formation d'un léger arrondi 6 (voir figure 5) créé par l'effondrement du métal, à leur partie inférieure la formation de bavures 7 par effet de cisaillement ainsi qu'une certaine rugosité de leurs flancs 8 de découpe.

En second lieu on donne au flan ajouré 2 une section transversale sensiblement en U présentant une base 9 arrondie et des ailes 10 (voir figures 2 et 4A) par une opération de pliage effectuée par une presse dont les poinçons et matrice sont conformés de façon à effectuer simultanément un léger embouti sur une partie seulement de la zone ajourée 3 de la grille pour former la zone active de celle-ci. Dans la réalisation préférée représentée aux figures 3A et 4A cet embouti a la forme d'un méplat 11 sensiblement rectangulaire.

L'opération suivante est une opération classique de trempe et de revenu de la grille préformée destinée à donner au métal ses caractéristiques mécaniques définitives.

Dans l'étape suivante on rectifie à la meule la face intérieure 12 du méplat embouti 11 et on polit par exemple au moyen d'un disque la face extérieure de la grille y compris la base 9 de l'U et le méplat 11.

L'opération de rectification entraîne la disparition des bavures de découpe 7 sur la face intérieure des travaux 5 mais crée d'autres bavures et du morfil 13 aux angles de la nouvelle face intérieure des barreaux 5 rectifiés (voir figure 6). Le polissage donne à la grille son brillant, adoucit les angles 6 et les flancs de découpe 8 des barreaux 5 et fait tomber une partie des bavures et du morfil 13.

Comme il ressort de la description ci-dessus, ce procédé présente plusieurs avantages importants.

Tout d'abord, l'emploi d'acier inoxydable à la place d'acier au carbone permet de supprimer deux opérations : la rectification de la face extérieure du méplat 11 et le chromage.

D'autre part, la dureté plus grande de l'acier inoxydable permet de partir d'une feuille métallique plus mince. Au total, bien qu'utilisant une matière première plus coûteuse, le bilan économique du procédé selon l'invention est positif.

D'autre part les performances de la tête de coupe sont améliorées.

En effet si la rectification de la face interne du méplat 11 crée des bavures et du morfil 13, n'étant pas chromés, ces bavures et ce morfil tomberont par action des couteaux dès les premières utilisations du rasoir, l'expérience montrant que les couteaux ne risquaient pas d'être endommagés par cette action. De même l'absence de chromage permettra d'obtenir un meilleur polissage des flancs 8 et des arrondis de découpe 6.

On va maintenant préciser certaines caractéristiques de la grille de rasoir électrique selon l'invention.

Dans la réalisation décrite à la figure 1 la feuille métallique de départ 1 est par exemple un acier de coutellerie inoxydable martensitique à 13% de chrome d'épaisseur 0,2 mm.

Le flan 2 de forme générale rectangulaire, porte deux rangées d'ouvertures 4 rectangulaires, étroites, transversales, à pas constant qui sont placées symétriquement par rapport à l'axe X-X et dont l'espacement 5 dans les rangées et entre rangées est du même ordre de grandeur que la largeur des ouvertures 4.

La zone 3 occupée par les ouvertures 4 n'occupe longitudinalement que la région centrale laissant pleines de part et d'autre deux bandes latérales 110 symétriques qui forment après pliage les ailes 10 de l'U, et les deux extrémités longitudinales 9a et 9b du flan 2.

Les ailes 10 de l'U comportent une partie 10a sensiblement parallèle et perpendiculaire au fond de cet U, suivie d'une partie 10b légèrement ouverte (voir figure 4A).

Le méplat 11 présente une forme sensiblement rectangulaire et n'occupe dans le sens de la largeur, qu'environ la moitié de la zone 3 des ouvertures de la grille tandis qu'il déborde cette zone longitudinalement pour présenter à ses extrémités deux cuvettes 14 non ajourées et deux pliures d'embouti 15 perpendiculaires à l'axe transversal de la grille (voir figures 2, 3A et 4A).

Le méplat 11 présente de préférence une faible profondeur d'embouti de l'ordre de grandeur de l'épaisseur de la feuille métallique de départ 1.

La rectification effectuée sur la face intérieure 12 du méplat 11 intéresse la totalité de celui-ci dans le sens longitudinal mais est transversalement égale à celle du fond de l'embouti pour former l'évidement 16 (voir figures 3B et 4B).

Cette rectification a pour objet de ramener l'épaisseur de la zone active de la grille à environ 0,07 mm épaisseur généralement admise comme procurant la meilleure efficacité à la tête de coupe pour un rasoir électrique.

Aux figures 4B et 6 on a représenté en pointillé un couteau 17 de forme semi-cylindrique présentant l'angle de coupe 19 pour coopérer avec la grille et s'inscrivant dans l'évidement 16. Son mouvement alternatif de va et vient assuré par tous moyens moteurs bien connus est symbolisé par une double flèche à la figure 6.

Complémentairement, la grille de rasoir présente de préférence des dimensions spécifiques; le rapport de la largeur totale de la tête qui est ici sensiblement confondue avec la base 9 de l'U à la largeur du méplat 11 est de préférence comprise entre 1,7 et 3. Dans la réalisation représentée ce rapport est de l'ordre de 2.

Par ailleurs les zones de raccordement 18 entre la base 9 de l'U et les parties 10a sensiblement parallèles des ailes 10 de cet U sont arrondies et présentent un rayon d'au moins 0,5 mm (voir figure 4B).

La structure de la grille conforme à l'invention dans laquelle la partie active amincie formée par le fond du méplat embouti 11, est encadrée sur chacun de ses côtés par une bande métallique pleine (10, 14, 9a, 9b) permet d'obtenir pour celle-ci une bonne rigidité malgré la faible épaisseur de la feuille métallique de départ 1 (0,2 mm).

Par ailleurs la forme générale de la grille en particulier le rayon des arrondis 18 des zones de raccordement entre la base 9 et les ailes 10 de l'U ainsi que le rapport entre la largeur totale de la tête 9 et la largeur du méplat 11 concourent à donner à l'utilisateur une sensation agréable, de confort et de douceur.

Bien entendu l'invention n'est pas limitée aux exemples que l'on vient de décrire et on peut apporter à ceux-ci de nombreuses modifications sans sortir du cadre de l'invention.

Ainsi les ouvertures rectangulaires 4 du flan 2 pourraient être remplacées par des ouvertures en nid d'abeilles.

Par ailleurs le méplat 11 pourrait être longitudinalement divisé en 2 zones égales séparées par une zone étroite non emboutie donc non rectifiée.

10

REVENDICATIONS

1. Procédé pour fabriquer une grille pour tête de rasoir électrique, dans lequel on réalise, par découpage dans une feuille en acier inoxydable, un flan (2) sensiblement rectangulaire présentant les ouvertures (4) de la grille, on plie de flan de façon à obtenir une grille de section transversale en U, les ouvertures (4) de la grille s'étendant sur la base de cette section, puis on rectifie et on polit la surface de la grille, caractérisé en ce qu'après pliage du flan (2), on rectifie la face intérieure (12) de la base comportant les ouvertures pour amener la grille à son épaisseur normale d'utilisation, et on polit directement la face extérieure de cette base, sans la rectifier ni la chromer.

2. Procédé conforme à la revendication 1, caractérisé en ce qu'on forme sur la face extérieure de la base de la grille pliée en U un méplat (11) embouti vers l'intérieur de la grille, on rectifie la face intérieure (12) de ce méplat embouti et on polit la face extérieure de la grille y compris la face extérieure du méplat embouti.

3. Procédé conforme à la revendication 2, caractérisé en ce que la face intérieure (12) du méplat est rectifiée de façon à réduire l'épaisseur de la grille dans la zone du méplat (11), notamment à environ 0,07mm.

4. Grille pour tête de rasoir électrique comprenant une feuille en acier inoxydable (2) sensiblement rectangulaire pliée de façon à présenter une section transversale sensiblement en U, la base de l'U de cette grille présentant des ouvertures (4), caractérisée en ce que cette base comporte une cavité (11) emboutie vers l'intérieur de la grille et présentant un fond plat, en ce que la face interne de ce fond (12) comporte un évidement rectifié (16) dont la largeur est adaptée à celle des couteaux (17), et en ce que la face extérieure de la grille, y compris celle de la cavité emboutie est uniquement polie.

5. Grille conforme à la revendication 4, caractérisée en ce que la largeur de l'évidement rectifié (16) est sensiblement égale à la largeur du fond de la cavité emboutie (11).

6. Grille conforme à l'une des revendications 4 ou 5, caractérisée en ce que les bords opposés (15) de la cavité emboutie sont situés entre les bords adjacents de la grille et les bords opposés des ouvertures de la grille.

7. Grille conforme à l'une des revendications 4, 5 ou 6, caractérisée en ce que le méplat (11) ne couvre en largeur qu'une partie des ouvertures (4) de la grille.

8. Grille conforme à l'une quelconque des revendications 4, 5, 6 ou 7, caractérisée en ce que la profondeur de la cavité emboutie (11) est sensiblement égale à l'épaisseur de la feuille métallique de départ (1).

9. Grille conforme à l'une quelconque des revendications 4 à 8, caractérisée en ce que le rayon de raccordement (18) entre la base (9) de la grille et ses ailes (10a) est au moins égal à 0,5 mm.

10. Grille conforme à l'une quelconque des revendications 4 à 9, caractérisée en ce que le rapport de la largeur totale de la base (9) de l'U formé par la grille sur la largeur de la zone active des ouvertures (4) de cette base est compris entre 1,7 et 3.

FIG.1

FIG.2

FIG.3A

FIG.3B

0108669

## FIG.4A

## FIG.4B

## FIG.5

## FIG.6

## Office européen des brevets

# RAPPORT DE RECHERCHE EUROPEENNE

Numéro de la demande

EP 83 40 1985

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | US-A-2 182 067 (BRUECKER)<br>* Page 3, colonne de gauche, lignes 1-45; page 4, colonne de gauche, lignes 1-5; figures 11,15,20 * | 1,2,4 | B 26 B 19/38<br>B 21 D 53/64 |
| | --- | | |
| Y | FR-A-2 263 840 (MATSUSHITA)<br>* Page 5, lignes 6-28; page 6, igne 31 - page 7, ligne 1; figures 3A-3E * | 1,2,4 | |
| | --- | | |
| Y | GB-A- 950 426 (SUNBEAM)<br>* Figure 3 * | 1,2,4 | |
| | --- | | |
| A | DE-A-1 813 617 (CARINTHIA)<br>* Page 4, ligne 22 - page 5, ligne 15; page 5, ligne 26; figures 1,2 * | 1,3,4 | |
| | --- | | **DOMAINES TECHNIQUES RECHERCHES (Int. Cl. ³)** |
| A | GB-A- 537 749 (MARTIN)<br>* Figure 1 * | 1,2,4 | B 26 B<br>B 21 D |
| | --- | | |
| A | CH-A- 375 625 (BRAUN)<br>* Page 2, lignes 85-92; figures 6,7 * | 1,4 | |
| | ----- | | |

Le présent rapport de recherche a été établi pour toutes les revendications

| Lieu de la recherche<br>LA HAYE | Date d'achèvement de la recherche<br>11-01-1984 | Examinateur<br>COLAS R.P. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82